Europäisches Patentamt

European Patent Office

Office européen des brevets

(19)

(11) Publication number: **0 340 174**
A2

# (12) EUROPEAN PATENT APPLICATION

(21) Application number: **89830101.5**

(22) Date of filing: **07.03.89**

(51) Int. Cl.⁴: **A 61 F 2/36**
**A 61 L 27/00**

(30) Priority: **29.04.88 IT 2040188**

(43) Date of publication of application:
**02.11.89 Bulletin 89/44**

(84) Designated Contracting States:
**AT BE CH DE ES FR GB GR LI NL SE**

(71) Applicant: **G. CREMASCOLI S.p.A.**
**Via Clemente Prudenzio, 14/16**
**I-20138 Milano (IT)**

(72) Inventor: **Cremascoli, Patrizio G. Cremascoli S.p.A.**
**Via Clemente Prudenzio, 14/16**
**I-20138-Milano (IT)**

(74) Representative: **Cicogna, Franco**
**Ufficio Internazionale Brevetti Dott.Prof. Franco Cicogna**
**Via Visconti di Modrone, 14/A**
**I-20122 Milano (IT)**

(54) Composite-material prosthesis femoral stem.

(57) The present invention relates to a composite-material prosthesis femoral stem, comprising a plurality of layers (3) made of kevlar or other like materials impregnated and/or coated with different particle size powders of alumina ($Al_2O_3$) or zirconium oxydes ($ZrO_2$) or hydroxylapatite $Ca_{10}(PO_4)_6(OH_2)$.

EP 0 340 174 A2

## Description

### COMPOSITE-MATERIAL PROSTHESIS FEMORAL STEM

The present invention relates to a composite-material femoral stem, having a suitably layered structure and adapted to be coated by different particle size powders of alumina, zirconium oxydes or hydroxylapatite.

As is known in order to recover the functionality of a human hip, there are presently used prostheses substantially consisting of femoral stems which are introduced into the femour of a patient so as to allow for this patient to gradually recover his/her articulation performance.

These prostheses are usually made of metal materials such as titanium and alloys thereof, which have been found to provide good mechanical characteristics and a good compatibility with the intended bone application.

These prostheses, however, are rather expensive and of complex construction.

Thus, a need exists of finding other types of prostheses which do not require the use of titanium and alloys thereof.

### SUMMARY OF THE INVENTION

Accordingly, the task of the present invention is to overcome the above mentioned drawbacks, by providing a stem, adapted to operate as a prosthesis for recovering bone tissues, which has an elastic modulus similar to that of the bone, while being perfectly biocompatible with the body tissues and provided with a high mechanical strength.

Within the scope of the above mentioned task, a main object of the present invention is to provide such a femoral stem which has mechanical characteristics nearly equivalent to those of the bone in which said stem is to be applied, jointly with a suitable flexibility or resiliency.

Another object of the present invention is to provide such a femoral stem which can be constructed with any required shape, can be easily fitted in the femoral bone and which, moreover, is very reliable in operation.

According to one aspect of the present invention, the above mentioned task and objects, as well as yet other objects, which will become more apparent hereinafter, are achieved by a femoral stem characterized in that said femoral stem consists of a layered structure of kevlar, the surface layer of which is impregnated with different particle size powders of alumina ($Al_2O_3$) or zirconium oxydes ($ZrO_2$), or hydroxylapatite $CA_{10}(PO_4)_6(OH_2)$.

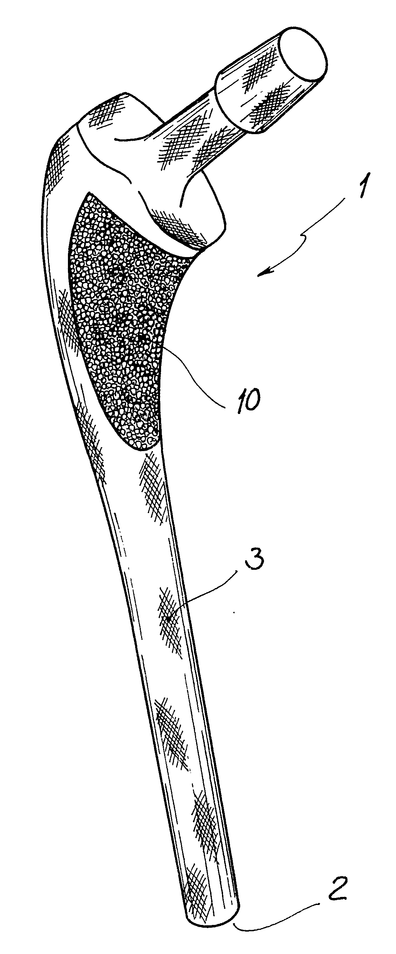

### BRIEF DESCRIPTION OF THE DRAWING

Further characteristics and advantages of the invention will become more apparent from the following detailed description of a preferred, though not exclusive, embodiment of a prosthesis femoral stem according to the invention which is shown, by way of an indicative but not limitative example, in the accompanying drawing the single figure of which schematically shows a possible embodiment of this femoral stem.

### DESCRIPTION OF THE PREFERRED EMBODIMENT

With reference to the drawing figure, the prosthesis femoral stem according to the invention, which is indicated overally at the reference number 1, substantially consists of an inner core of elongated shape, indicated at 2, which can be made of any suitable thermosetting plastics material. Alternatively, this core can be formed by a metal material tubular element or a combination of a plastics material reinforced by a metal core or as a single composite layered structure.

The main feature of the invention is that the femoral stem is coated, at the top thereof, by a plurality of layers 3, made of kevlar, which can be suitably impregnated with different particle size powders of alumina ($Al_2O_3$), so as to provide the desired me chanical stiffness.

If desired. it is also possible to apply a coating made of zirconium oxydes ($ZrO_2$), adapted to provide a surface layer of even and compact structure.

According to a further embodiment, the coating can be made by impregnating the kevlar layer with hydroxylapatite $Ca_{10}(PO_4)_6(OH_2)$.

If desired, moreover, selected zones of the layered structure can be filled by the same impregnating plastics materials has it is schematically shown at 10 in the drawing.

Thus, owing to the disclosed method, the femoral stem can be shaped to the desired configuration in a very simple and quick way.

Moreover, the composite material structure of this femoral stem provides the desired resiliency characteristics, jointly with the necessary mechanical strength, thus providing a very efficient prosthesis femoral stem effective to accelerate the recovering of the encompassing bone tissues.

From the above disclosure it should be apparent that the invention fully achieves the intended task and objects.

While the invention has been disclosed and illustrated with reference to a preferred embodiment thereof, it should be apparent that the disclosed embodiment is susceptible to several modifications and variations all of which will come within the spirit and scope of the appended claims.

### Claims

1. A femoral stem, characterized in that said femoral stem consists of a layered structure of kevlar the surface layer of which is impregnated with different particle size powders of alumina ($Al_2O_3$).

2. A femoral stem, characterized in that said femoral stem comprises a core at least partially coated by a coating consisting of a layered structure made of kevlar impregnated with zirconium oxyde ($ZrO_2$).

3. A femoral stem, characterized in that said femoral stem comprises a core which is at least partially coated by a coating consisting of a layered structure of kevlar impregnated with hydroxylapatite $Ca_{10}(PO_4)_6(OH_2)$.

4. A femoral stem, according to one or more of the preceding claims, characterized in that said femoral stem further comprises, inside said core, a metal material reinforcement element.

5. A femoral stem, according to one or more of the preceding claims, characterized in that said femoral stem comprises filler materials at regions not covered by said layered structure.

6. A femoral stem, according to one or more of the preceding claims, characterized in that said femoral stem comprises a layered structure of kevlar impregnated with binding resins preferably of the epoxydic type.

7. A femoral stem, according to one or more of the preceding claims, characterized in that the composite material forming said stem is so designed and arranged as to facilitate the recovering of the bone tissue on the prosthesis surface of said stem.